# EUROPEAN PATENT APPLICATION

(11) **EP 4 300 404 A1**
(43) Date of publication of application: **03.01.2024**
(21) Application number: 23180139.0
(22) Date of filing: 19.06.2023
(51) Int. Cl.: G06Q 30/0601

(54) **SHOE PRODUCT INFORMATION PROVIDING DEVICE AND SHOE PRODUCT INFORMATION PROVIDING METHOD**

(30) Priority: 30.06.2022 JP 2022106513
(71) Applicant: ASICS Corporation, Kobe-shi Hyogo 650-8555 (JP)
(72) Inventor: Nakaya, Mai, Hyogo, 650-8555 (JP); Kusumi, Hiroyuki, Hyogo, 650-8555 (JP); Shiina, Ippei, Hyogo, 650-8555 (JP); Kusano, Ken, Hyogo, 650-8555 (JP)
(74) Representative: Marks & Clerk LLP

(57) **Abstract**

A shoe purchase recommendation device 10A includes a user information acquisition unit 18 that acquires user information 111 including a first foot size 111B that is a foot size of a user, a foot-size prediction unit 12 that predicts, based on the first foot size 111B of the user, a second foot size that is a foot size of the user at a future point of time, a growth-end determination unit 16 that determines, based on the second foot size, whether foot growth of the user has ended at the future point of time, a product information storage unit 13 that stores child product information 131 and adult product information 132, a product information selection unit 14 that selects, based on a result determined by the growth-end determination unit 16, product information regarding shoes from one of the child product information 131 and the adult product information 132, and a display processing unit 15 that displays the product information regarding shoes selected by the product information selection unit 14 on a display unit 21.

## Description

### CROSS-REFERENCE TO RELATED APPLICATIONS

The present application claims priority to and incorporates by reference the entire contents of Japanese Patent Application No. 2022-106513 filed in Japan on June 30, 2022.

### BACKGROUND OF THE INVENTION

### 1. Field of the Invention

The present invention relates to a shoe product information providing device and a shoe product information providing method that predict a change in a foot size of a user and provide product information regarding shoes.

### 2. Description of the Related Art

It is necessary to wear shoes with an appropriate foot size including the foot length, the foot circumference, and the arch height of a user, and wearing shoes that are not suitable for the foot size causes hallux valgus or shoe sores.

The foot size changes with aging, but the size does not change equally at each part of a foot. Thus, the foot shape also changes as the foot size changes with aging. For this reason, it is known that the foot shape of children differs from that of adults. Generally, the foot shape of children is a square type with little difference in the length of each toe, and as they grow, the foot shape changes to an oblique or round type with a difference in the length of each toe. In addition, there are individual differences in the final foot size at the end of growth, and even if the foot size is the same, the foot shape can be that of children or adults. For example, for those with a large final foot length, a foot with a foot length of 22 cm has the foot shape of a growing child, and for those with a small final foot length, a foot with a foot length of 22 cm has the foot shape of an adult whose growth has ended.

Lasts used in manufacturing shoes have different shapes for children and adults. That is, shoes for children are manufactured using lasts suitable for foot shapes of children, and shoes for adults are manufactured using lasts suitable for foot shapes of adults. In addition, materials of parts can be different between shoes for children and shoes for adults, and adult shoes are generally made of stiffer and heavier materials.

Since the size ranges of products for children partially overlap with the size ranges of products for adults, in the transition period of growth, shoes suitable for a foot size of a user can be found in both a child product group and an adult product group. At this time, if a user with a child foot shape purchases shoes for adults, or if a user with an adult foot shape purchases shoes for children, the shoes does not hold the feet sufficiently and easily fall off or cause shoe sores. Furthermore, if the shoes are too stiff, too soft, or too heavy for the user, it can cause the user to show their performance insufficiently.

Therefore, when purchase of shoes is recommended, it is necessary to provide product information regarding shoes in consideration of the foot shape of the user, such as a foot shape of children or a foot shape of adults whose growth have ended.

WO 2021/130889 A discloses an invention that predicts a change in foot length and notifies a customer of a replacement of shoes.

The invention disclosed in WO 2021/130889 A can provide product information regarding shoes suitable for the foot length of a user, but cannot provide product information regarding shoes in consideration of whether the foot shape of the user is an adult foot shape.

The present invention has been made in view of the above, and a purpose of the present invention is to obtain a shoe product information providing device capable of providing product information regarding shoes in consideration of whether a foot shape of a user is an adult foot shape.

### SUMMARY OF THE INVENTION

In order to solve the above problem and achieve the object, a shoe product information providing device comprising: a user information acquisition unit configured to acquire user information including a first foot size, the first foot size being a foot size of a user; a user information storage unit configured to store the user information acquired by the user information acquisition unit; a foot-size prediction unit configured to predict, based on the first foot size of the user stored in the user information storage unit, a second foot size, the second foot size being a foot size of the user at a future point of time; a growth-end determination unit configured to determine, based on the second foot size predicted by the foot-size prediction unit, whether foot growth of the user has ended at the future point of time; a product information storage unit configured to store child product information, the child product information being information regarding a product group of shoes for children, and adult product information, the adult product information being information regarding a product group of shoes for adults; a product information selection unit configured to select, based on a result determined by the growth-end determination unit, product information regarding shoes from one of the child product information and the adult product information; and a display processing unit configured to display the product information regarding shoes selected by the product information selection unit on a display unit.

### BRIEF DESCRIPTION OF THE DRAWINGS

FIG. 1 is a diagram illustrating a configuration of a shoe purchase recommendation device according to a first embodiment of the present invention;
FIG. 2 is a diagram illustrating an example of a purchase recommendation screen displayed by a screen display processing unit of the shoe purchase recommendation device according to the first embodiment;
FIG. 3 is a flowchart illustrating an operation procedure of the shoe purchase recommendation device according to the first embodiment;
FIG. 4 is a diagram illustrating an example of a relationship between user age and foot circumference ratio;
FIG. 5 is a diagram illustrating a configuration of a shoe purchase recommendation device according to a second embodiment of the present invention;
FIG. 6 is a diagram illustrating a configuration of a shoe purchase recommendation device according to a third embodiment of the present invention;
FIG. 7 is a diagram illustrating an example of a last conversion table of a product information selection unit according to the third embodiment;
FIG. 8 is a diagram illustrating a configuration of a shoe purchase recommendation device according to a fourth embodiment of the present invention;
FIG. 9 is a flowchart illustrating a procedure of operation in which the shoe purchase recommendation device according to the fourth embodiment selects product information regarding shoes with a foot circumference size one-step closer to a standard size than the size suitable for the foot circumference of a user at a future point of time; and
FIG. 10 is a diagram illustrating a hardware configuration example of the shoe purchase recommendation device according to the first to fourth embodiments.

### DETAILED DESCRIPTION OF THE PREFERRED EMBODIMENTS

Hereinafter, embodiments of a shoe product information providing device and a shoe product information providing method according to the present invention will be described in detail with reference to the drawings. Note that the present invention is not limited by the embodiments. In the following embodiments, the same or common parts are denoted by the same reference signs, and the description thereof will not be repeated.

In the following description, a case where a foot size means a foot length and a foot circumference is described as an example, but the foot size may include a dimension of a measurable part, such as an arch height. The foot size may also include three-dimensional scan data on a foot of a user. In addition, the foot size is not only the size of the foot itself, but also the dimensions of the parts of a shoe corresponding to the foot length, the foot circumference, the arch height, and the like may be regarded as the foot size.

### First embodiment

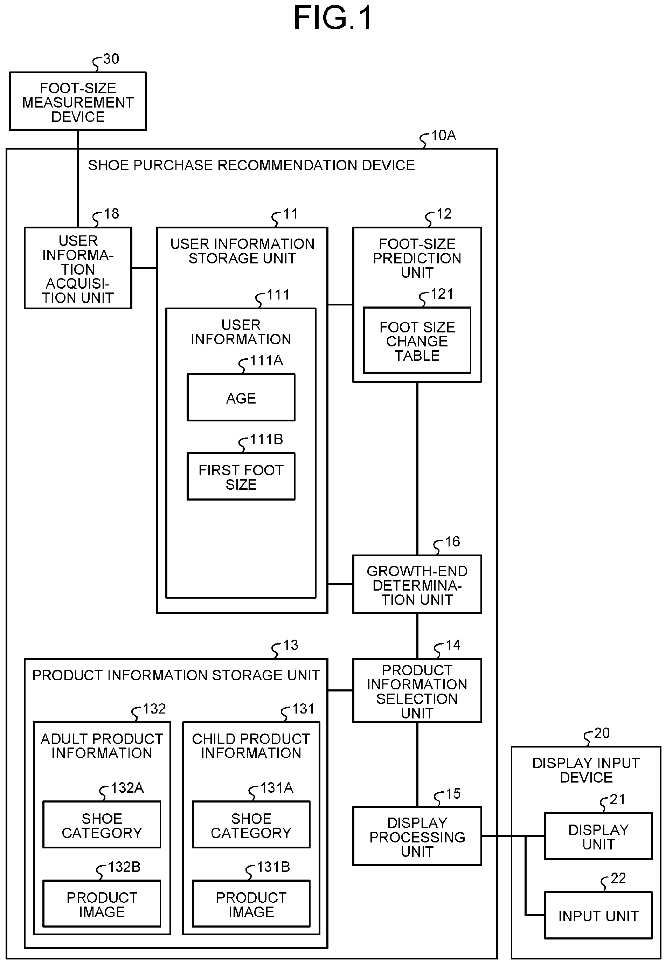

FIG. 1 is a diagram illustrating a configuration of a shoe purchase recommendation device according to a first embodiment of the present invention. A shoe purchase recommendation device 10A is an embodiment of a shoe product information providing device that provides a user with product information regarding shoes, and provides a user with product information regarding shoes to be recommended for purchase. The shoe purchase recommendation device 10A includes a user information acquisition unit 18, a user information storage unit 11, a foot-size prediction unit 12, a product information storage unit 13, a growth-end determination unit 16, a product information selection unit 14, and a display processing unit 15. The user information acquisition unit 18 acquires user information including a first foot size that is a foot size of a user. The user information storage unit 11 stores user information 111 including at least an age 111A and a first foot size 111B of the user. The foot-size prediction unit 12 predicts, based on the first foot size 111B, a second foot size that is a foot size of the user at a future point of time. The product information storage unit 13 stores child product information 131 and adult product information 132. The growth-end determination unit 16 determines, based on the second foot size predicted by the foot-size prediction unit 12, whether the foot growth of the user has ended at the future point of time. The product information selection unit 14 selects, based on a result determined by the growth-end determination unit 16, product information regarding shoes from one of the child product information 131 and the adult product information 132. The display processing unit 15 displays the product information regarding shoes selected by the product information selection unit 14. The first foot size 111B is data on the foot size of the user at the present and a past point of time. The age 111A is data representing the elapsed time from birth. The age 111A may be "age in month" representing the elapsed time from birth in months instead of years. The child product information 131 is information regarding a product group of shoes for children and includes a shoe category 131A and a product image 131B. The adult product information 132 is information regarding a product group of shoes for adults, and includes a shoe category 132A and a product image 132B. The first foot size and the second foot size each mean the foot length and the foot circumference of the user.

The shoe categories 131A and 132A are information indicating categories of shoes. The categories include sports shoes for less lateral movement such as those for running and trail running, sports shoes for lateral movement such as those for basketball and volleyball, and general sports shoes not specialized in a specific sport. Note that the categories mentioned here are examples, and shoes may be classified in categories different from those examples.

The foot-size prediction unit 12 retains a foot size change table 121, which is statistical data on a change in foot size, extracts an average foot size change amount, which is an average change amount of the foot size for each age, from the foot size change table 121 based on the age 111A, and calculates the second foot size by adding the extracted average foot size change amount to the first foot size 111B as a predicted foot size change amount. The average foot size change amount can be calculated by extracting the amount of change in foot size at the target age of a sample of the same gender as the user and taking the average of a plurality of samples.

For example, the second foot size in the birthday month in (X + 1) years old is calculated as the sum of the first foot size and the predicted foot size change amount from the present to the birthday month in (X + 1) years old. Therefore, if the current age is X years old and the number of elapsed months from the birth month is Y months, the predicted foot size change amount from the present to the birth month in (X + 1) years old is (average foot size change amount in (X + 1) years old) × (12 - Y)/12. Similarly, the second foot size in the birth month in (X + 2) years old is calculated as the sum of the second foot size in the birth month in (X + 1) years old and the average foot size change amount in (X + 2) years old.

Note that, if at least one of the weight of the user, the size of the user, the presence or absence of growth pain of the user, the size of a parent of the user, the height of a parent of the user, and the growth peak time of a parent of the user is known, the foot-size prediction unit 12 may correct the second foot size based on these pieces of information. For example, if the weight or the size of the hand of the user is less than the average value of the same age group, a change in foot size is expected to be small in the future, and thus the foot-size prediction unit 12 can add a negative correction to the second foot size. If the user has growth pain, the foot size is expected to greatly increase in the future, and thus the foot-size prediction unit 12 can add a positive correction to the second foot size. If the current age of the user exceeds the growth peak age of a parent of the user, a change in foot size is expected to be small in the future, and thus the foot-size prediction unit 12 can add a negative correction to the second foot size. In addition, since the growth peak of the user is expected to be around the growth peak age of a parent of the user, if the age of the user is close to the growth peak age of the parent of the user, the foot-size prediction unit 12 can add a positive correction to the second foot size.

The shoe purchase recommendation device 10A is connected to a display input device 20 including a display unit 21 and an input unit 22. To the display unit 21, a liquid crystal display or an organic electroluminescence display can be applied, but a display device other than these may be applied. To the input unit 22, an input device, such as a keyboard or a pointing device, can be applied, but an input device other than these may be applied. Note that a touch panel having both a display function and an input function can also be applied to the display unit 21 and the input unit 22. The display input device 20 is connected to the shoe purchase recommendation device 10A via the Internet. Note that the display input device 20 may be directly connected to the shoe purchase recommendation device 10A without the Internet. Alternatively, the shoe purchase recommendation device 10A may include the display unit 21 and the input unit 22. In that case, it is not necessary to connect the display input device 20 to the shoe purchase recommendation device 10A.

The shoe purchase recommendation device 10A is connected to a foot-size measurement device 30. The foot-size measurement device 30 outputs the first foot size 111B obtained by measuring the foot size of the user. The first foot size 111B is accumulated in the user information storage unit 11. Note that a user interface for input may be provided in the shoe purchase recommendation device 10A, and the first foot size 111B may be input by operating the user interface. Alternatively, the shoe purchase recommendation device 10A may acquire the first foot size 111B by communicating with another device that stores the first foot size 111B.

As an example, a server of a shoe manufacturer can be applied to the shoe purchase recommendation device 10A. In addition, a smartphone terminal of the user can be applied to the display input device 20. Furthermore, a three-dimensional foot-shape automatic measuring instrument or an information terminal, such as a smartphone terminal and a tablet terminal of the user, can be applied to the foot-size measurement device 30.

Shoes have a foot size fitting range for each shoe size. As an example, for shoes with a foot length size of 20 cm, a foot length of 19.15 cm to 19.56 cm is set as the fitting range. In addition, shoes are varied in size with a plurality of different foot circumference sizes for the same foot length size. As an example, shoes with a foot length of 20 cm are varied in size with three foot circumference sizes of "E", "EE", and "EEE". At this time, a foot circumference of 213 mm to 218 mm is set as the fitting range for shoes with the foot circumference size "E". In addition, a foot circumference of 219 mm to 224 mm is set as the fitting range for shoes with the foot circumference size "EE". In addition, a foot circumference of 225 mm to 230 mm is set as the fitting range for shoes with the foot circumference size "EEE".

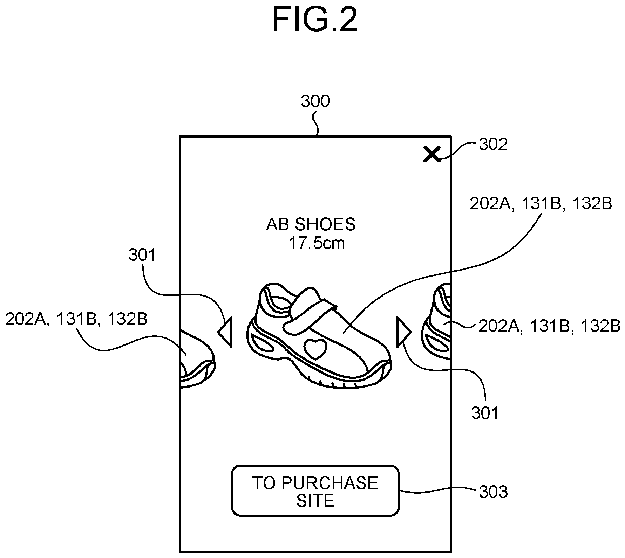

FIG. 2 is a diagram illustrating an example of a purchase recommendation screen displayed by a screen display processing unit of the shoe purchase recommendation device according to the first embodiment. A purchase recommendation screen 300 is a screen that displays the product information regarding the shoes selected by the product information selection unit 14. The purchase recommendation screen 300 includes a purchase recommendation product image 202A. The purchase recommendation product image 202A is the product image 131B or 132B of the purchase recommendation product. If the product information selection unit 14 selects a plurality pieces of product information regarding shoes, the product image 131B or 132B of the shoes with the highest recommendation priority is displayed at the center, and the product image 131B or 132B of the shoes with priority other than the highest recommendation priority is displayed such that only a part of the product image appears in the purchase recommendation screen 300. For example, the product image 131B or 132B of the shoes with priority other than the highest recommendation priority is displayed such that only the toe or only the heel appears in the purchase recommendation screen 300. In this manner, when the product information regarding shoes selected by the product information selection unit 14 includes product information regarding a first pair of shoes and product information regarding a second pair of shoes, which is different in type from the first pair of shoes, the display processing unit 15 displays the product information regarding the first pair of shoes and the product information regarding the second pair of shoes selected by the product information selection unit 14 on the display unit 21.

When the product information selection unit 14 selects a plurality of pieces of product information regarding shoes, a switch button 301 is displayed on the purchase recommendation screen 300. When the switch button 301 is pressed by an operation to the input unit 22, the display processing unit 15 switches the product image 131B or 132B displayed at the center of the purchase recommendation screen 300. Therefore, the user of the display input device 20 can display the entire product image 131B or 132B of shoes with priority other than the highest recommendation priority at the center of the purchase recommendation screen 300 by pressing the switch button 301 by operating the input unit 22. Note that, if the number of pieces of product information regarding shoes to be displayed on the purchase recommendation screen 300 is too large, it becomes difficult for the user to determine which shoes to purchase. Therefore, the number of pieces of product information regarding shoes to be displayed on the purchase recommendation screen 300 is preferably five or less. For example, in the shoe purchase recommendation device 10A according to the first embodiment, by setting the number of pieces of product information regarding shoes to be displayed on the purchase recommendation screen 300 to three, at least a part of the product image 131B or 132B of each shoe is displayed on the purchase recommendation screen 300, which allows the user to easily determine which shoes to purchase.

In addition, a link 303, which is a uniform resource locator, is displayed on the purchase recommendation screen 300. By pressing the link 303 by operating the input unit 22, the user of the display input device 20 can purchase the shoes whose product image 131B or 132B is displayed at the center of the purchase recommendation screen 300 on an electronic commerce site.

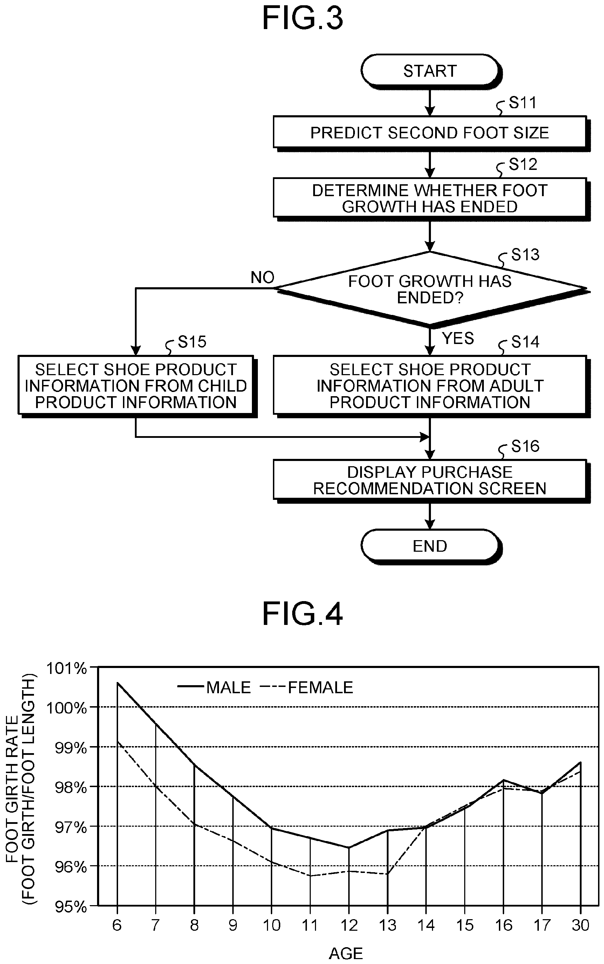

FIG. 3 is a flowchart illustrating an operation procedure of the shoe purchase recommendation device according to the first embodiment. In step S11, the foot-size prediction unit 12 predicts the second foot size, which is the foot size of the user at a future point of time, based on the first foot size 111B stored in the user information storage unit 11. In step S12, the growth-end determination unit 16 determines whether the foot growth of the user has ended at the future point of time based on the second foot size predicted by the foot-size prediction unit 12. Specifically, the growth-end determination unit 16 determines that the foot growth of the user has ended at the future point of time when both of a first condition that a foot circumference ratio, which is a value obtained by dividing the foot circumference of the user at the future point of time by the foot length, is greater than or equal to a preset threshold and a second condition that the age of the user at the future point of time is greater than or equal to a predetermined age are satisfied. The growth-end determination unit 16 determines that the foot growth of the user has not ended at the future point of time when at least one of the first condition and the second condition is not satisfied.

FIG. 4 is a diagram illustrating an example of a relationship between user age and foot circumference ratio. Specifically, the graph illustrated in FIG. 4 is derived by measuring foot sizes, including the foot circumference and the foot length, of a plurality of male and female subjects in each age group, and calculating the average value from the measurement results. Note that, foot circumference ratio = foot circumference/foot length. As illustrated in the graph in FIG. 4, it is known that the foot circumference ratio decreases from early childhood to pre-teens and then begin to increase. Therefore, after a change in foot circumference ratio stops decreasing at a certain age, the foot circumference ratio increases. On average, the age at which a change in foot circumference ratio stops decreasing is 11 years old. In addition, a change in foot circumference ratio stops decreasing at about 96%, and the foot circumference ratio in an adult foot shape is 98% or more. Therefore, as an example, when the age of the user is 11 years old or older and the foot circumference ratio is 98% or more, the growth-end determination unit 16 determines that both the first condition and the second condition are satisfied and determines that the foot growth of the user has ended. Note that, the second condition is that the age of the user at the future point of time is greater than or equal to the predetermined age in this description, but the second condition may be that the age in month of the user at the future point of time is greater than or equal to a predetermined age in month.

When the growth-end determination unit 16 determines that the foot growth of the user has ended, which means "Yes" in step S13, and the processing proceeds to step S14. When the growth-end determination unit 16 determines that the foot growth of the user has not ended, which means "No" in step S13, and the processing proceeds to step S15.

In step S14, the product information selection unit 14 selects product information regarding shoes from the adult product information 132. Specifically, the product information selection unit 14 selects product information regarding shoes with a shoe size suitable for the second foot size from the adult product information 132. The product information selection unit 14 may select product information regarding shoes with a shoe size that allows for a margin of about 0.5 cm to 1 cm with respect to the second foot size. If selecting a plurality of pieces of product information regarding shoes with a shoe size suitable for the second foot size, the product information selection unit 14 selects product information regarding shoes classified in different categories. For example, when selecting two pieces of product information regarding shoes, the product information selection unit 14 selects product information regarding shoes in a running category and product information regarding shoes in a general athletic shoe category. In an example in which the product information selection unit 14 selects two pieces of product information regarding shoes of a first pair of shoes and a second pair of shoes, the first pair of shoes is classified in a category for running, and the second pair of shoes is classified in a category for general athletic shoes different in use from running. Note that the product information selection unit 14 may select product information regarding a first pair of shoes and product information regarding a second pair of shoes from product information regarding shoes in the same category. In this case, product information regarding shoes of the same product and different colors may be selected as the product information regarding the first pair of shoes and the product information regarding the second pair of shoes. Alternatively, the product information selection unit 14 may select product information regarding shoes of different products as product information regarding a first pair of shoes and product information regarding a second pair of shoes.

In step S15, the product information selection unit 14 selects product information regarding shoes from the child product information 131. Specifically, the product information selection unit 14 selects product information regarding shoes with a shoe size suitable for the second foot size from the child product information 131. The product information selection unit 14 may select product information regarding shoes with a shoe size that allows for a margin of about 0.5 cm to 1 cm with respect to the second foot size. Note that, if selecting a plurality of pieces of product information regarding shoes with a shoe size suitable for the second foot size, the product information selection unit 14 selects product information regarding shoes classified in different categories.

In step S16, the display processing unit 15 displays the purchase recommendation screen 300.

Note that, if there is no product information regarding shoes with a shoe size suitable for the second foot size, the product information selection unit 14 notifies the display processing unit 15 that there are no shoes suitable for the foot length and the foot circumference of the user at the future point of time.

The display processing unit 15 notified that there are no shoes suitable for the foot length and the foot circumference of the user at the future point of time displays a message indicating that there are no shoes suitable for the foot length and the foot circumference of the user at the future point of time on the purchase recommendation screen 300.

The shoe purchase recommendation device 10A according to the first embodiment determines whether the foot growth of the user has ended, selects product information regarding shoes from the adult product information 132 when the foot growth has ended, and selects product information regarding shoes from the child product information 131 when the foot growth has not ended. Therefore, the shoe purchase recommendation device 10A according to the first embodiment can provide the user with product information regarding shoes manufactured using the last suitable for the foot shape of the user at the future point of time.

In the above, it has been described that the growth-end determination unit 16 determines that the foot growth of the user has ended at a future point of time when both of a first condition that a foot circumference ratio, which is a value obtained by dividing the foot circumference of the user at the future point of time by the foot length, is greater than or equal to a preset threshold and a second condition that the age of the user at the future point of time is greater than or equal to a predetermined age are satisfied, and determines that the foot growth of the user has not ended at the future point of time when at least one of the first condition and the second condition is not satisfied. However, the growth-end determination unit 16 may consider a third condition to determine whether the foot growth of the user has ended at the future point of time. The third condition is that the foot circumference ratio tends to increase at the future point of time.

Since there are individual differences in a change in foot circumference ratio, the foot circumference ratio for some users do not fall below 98% even in a period in which the foot circumference ratio decreases from early childhood to pre-teens. If it is determined whether the foot growth of such a user has ended based on only the first condition and the second condition, it is determined that the foot growth has ended if the age of the user is 11 years old or older even though the foot circumference ratio tends to decrease. However, in this case, the foot circumference ratio tends to decrease even when the age of the user is 11 years old or older, and thus it is preferable to determine that the foot growth has not ended. By determining whether the foot growth of the user has ended at the future point of time in consideration of the third condition that the foot circumference ratio tends to increase, the growth-end determination unit 16 can determine that the foot growth has not ended when the foot circumference ratio tends to decrease regardless of the age of the user. Therefore, by the growth-end determination unit 16 determining whether the foot growth of the user has ended at the future point of time in consideration of the third condition, it is possible to provide the user whose foot growth has not ended and feet are growing with product information regarding shoes selected from the child product information 131.

Furthermore, to perform the determination in consideration of the third condition, it is preferable to predict the second foot size, which is the foot size of the user at the future point of time, as accurately as possible. For this reason, the foot size of at least one of the father and mother of the user may be included in the user information 111, and the foot-size prediction unit 12 may predict the second foot size in consideration of the foot size of at least one of the father and mother of the user. It is known that the foot size of the user correlates with the foot sizes of the father and mother of the user. For example, if the foot size of at least one of the father and mother of the user is greater than or equal to the average value, the foot size of the user is also likely to be greater than or equal to the average value. Therefore, by including the foot size of at least one of the father and mother of the user in the user information 111 in order for the foot-size prediction unit 12 to predict the second foot size in consideration of the foot size of at least one of the father and mother of the user, it is possible to more accurately predict the second foot size, which allows the growth-end determination unit 16 to easily accurately determine whether the foot circumference ratio of the user tends to increase or decrease.

### Second embodiment

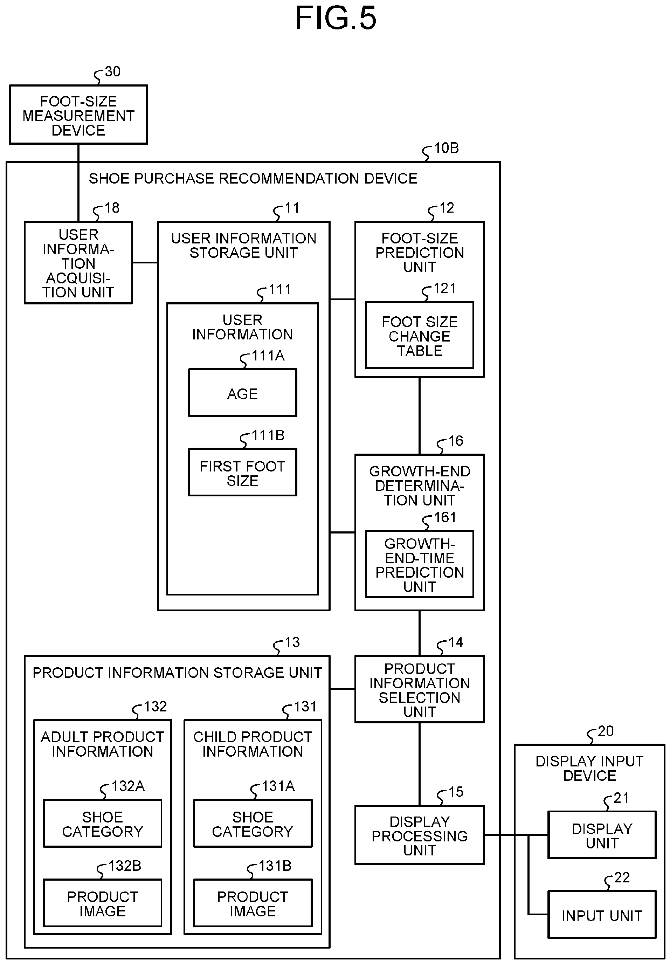

FIG. 5 is a diagram illustrating a configuration of a shoe purchase recommendation device according to a second embodiment of the present invention. A shoe purchase recommendation device 10B is different from the shoe purchase recommendation device 10A according to the first embodiment in that a growth-end determination unit 16 includes a growth-end-time prediction unit 161 that predicts the age at which the foot growth of a user ends based on a first foot size 111B stored in a user information storage unit 11. In the shoe purchase recommendation device 10B according to the second embodiment, the growth-end determination unit 16 determines that the foot growth of the user has ended at a future point of time when both of a first condition that a foot circumference ratio, which is a value obtained by dividing the foot circumference of the user at the future point of time by the foot length, is greater than or equal to a preset threshold and a second condition that the age of the user is greater than or equal to an age predicted by the growth-end-time prediction unit 161 at which the foot growth of the user ends are satisfied. The growth-end determination unit 16 determines that the foot growth of the user has not ended at the future point of time when at least one of the first condition and the second condition is not satisfied.

As illustrated in FIG. 4, before a change in foot circumference ratio stops decreasing, the decrease in foot circumference ratio becomes progressively smaller. Therefore, it is possible to predict the time when the change in foot circumference ratio stops decreasing based on the change in the decrease in foot circumference ratio. In the shoe purchase recommendation device 10B according to the second embodiment, although there are individual differences in the time when the change in foot circumference ratio stops decreasing, the growth-end-time prediction unit 161 predicts the time when the change in foot circumference ratio of the user stops decreasing based on the first foot size 111B stored in the user information storage unit 11, and it is possible to more accurately determine whether a growth in foot of the user has ended, as compared with the shoe purchase recommendation device 10A according to the first embodiment. Therefore, the shoe purchase recommendation device 10B can improve the accuracy of providing product information regarding shoes suitable for the foot of the user.

### Third embodiment

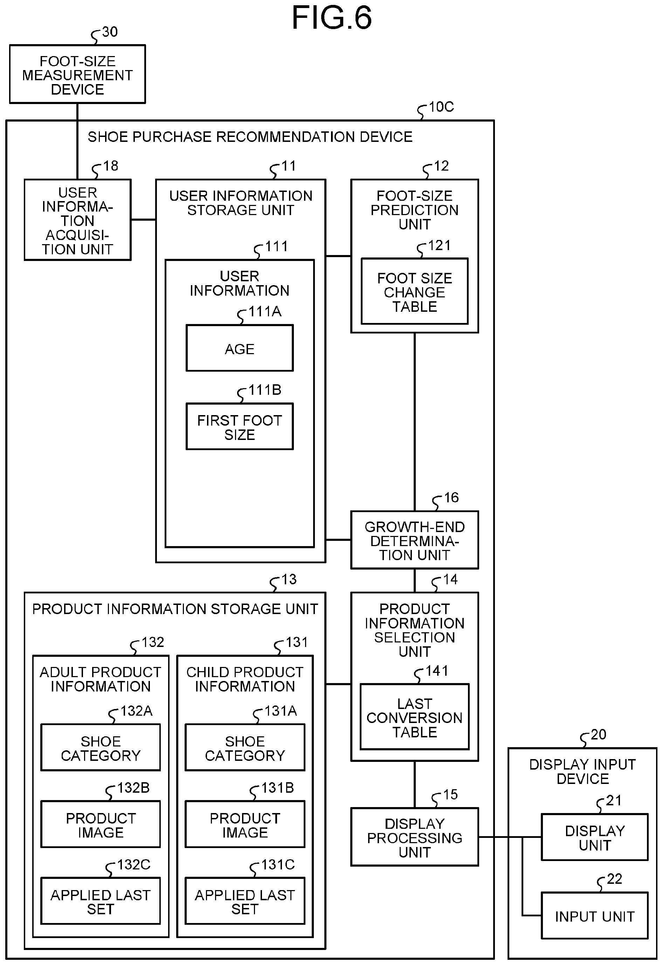

FIG. 6 is a diagram illustrating a configuration of a shoe purchase recommendation device according to a third embodiment of the present invention. In a shoe purchase recommendation device 10C according to the third embodiment, child product information 131 includes an applied last set 131C. In addition, adult product information 132 includes an applied last set 132C. The applied last sets 131C and 132C are information regarding last sets used in manufacturing shoes.

In the shoe purchase recommendation device 10C according to the third embodiment, a product information selection unit 14 includes a plurality of last conversion tables 141 indicating the correspondence between foot size and shoe size. The product information selection unit 14 selects product information regarding shoes from each of a plurality of models of shoes included in each of a plurality of categories, using the last conversion table 141 that matches the last set used for size variations.

Specifically, the product information selection unit 14 selects the last suitable for a second foot size in each of a plurality of last sets.

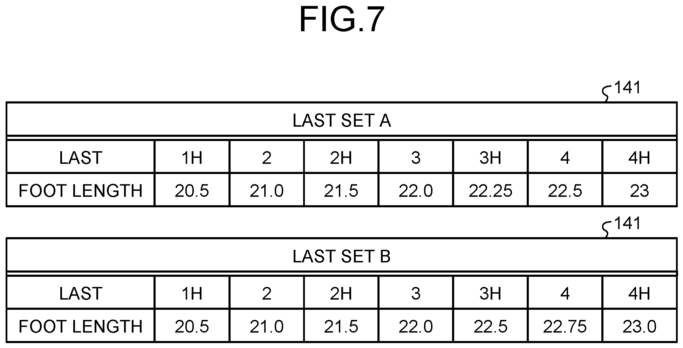

FIG. 7 is a diagram illustrating an example of a last conversion table provided in the product information selection unit according to the third embodiment. For the sake of simplicity of explanation, the table for foot length is only illustrated here, but the actual last conversion table 141 is a table indicating correspondence between the foot length and foot circumference and the suitable last. In this example, the product information selection unit 14 includes two last conversion tables 141 for a last set A and a last set B. However, the product information selection unit 14 may include three or more last conversion tables 141. The last set A and the last set B differ in the difference between last sizes in the last set. For this reason, for the same foot length, the shoes manufactured using the last set A and the shoes manufactured using the last set B can differ in the suitable size. For example, when the foot length of a user is 21.0 cm, the last suitable for the foot length of the user is the last "2" in both the last set A and the last set B. Meanwhile, when the foot length of a user is 22.5 cm, the last suitable for the foot length of the user is the last "4" in the last set A, and the last suitable for the foot length of the user is the last "3H" in the last set B.

The product information selection unit 14 identifies the last suitable for the second foot size in each of the plurality of last conversion tables 141, refers to the applied last set 131C or 132C included in the child product information 131 or the adult product information 132, and selects product information regarding shoes manufactured using the last suitable for the second foot size. For example, when the foot length of a user at a future point of time is predicted to be 22.5 cm, the product information selection unit 14 identifies the last "4" in the last set A and the last "3H" in the last set B as the last suitable for the second foot size. Then, the product information selection unit 14 selects product information regarding the shoes manufactured using the last "4" among the shoes of the model with size variations using the last set A and product information regarding the shoes manufactured using the last "3H" among the shoes of the model with size variations using the last set B.

The shoe purchase recommendation device 10C according to the third embodiment identifies the last suitable for the second foot size in each of the plurality of last sets, and selects product information regarding the shoes manufactured using the last suitable for the second foot size from the child product information 131 or the adult product information 132. Therefore, it is possible to provide product information regarding shoes suitable for the second foot size without being affected by the difference in shoe sizes due to the difference in lasts used to manufacture shoes.

### Fourth embodiment

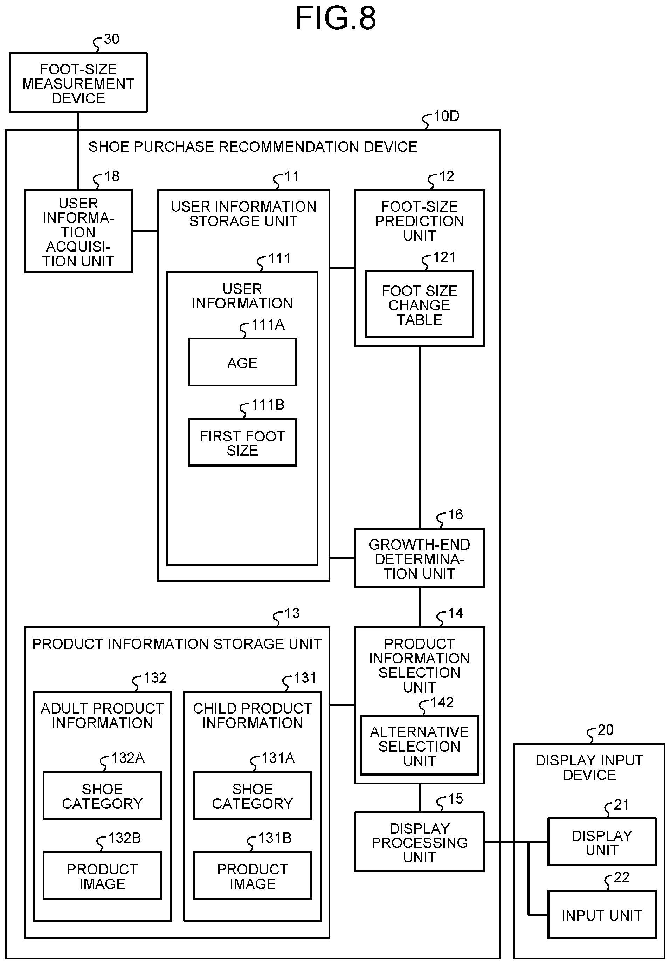

FIG. 8 is a diagram illustrating a configuration of a shoe purchase recommendation device according to a fourth embodiment of the present invention. A shoe purchase recommendation device 10D according to the fourth embodiment is different from the shoe purchase recommendation device 10A according to the first embodiment in that a product information selection unit 14 includes an alternative selection unit 142 that selects, when there is no product information regarding shoes suitable for a second foot size in child product information 131 and adult product information 132, product information regarding shoes suitable for the foot length of a user at a future point of time and having a foot circumference size one-step closer to a standard size than a size suitable for the foot circumference of the user at the future point of time. In the fourth embodiment, the second foot size includes the foot length and the foot circumference of the user at the future point of time.

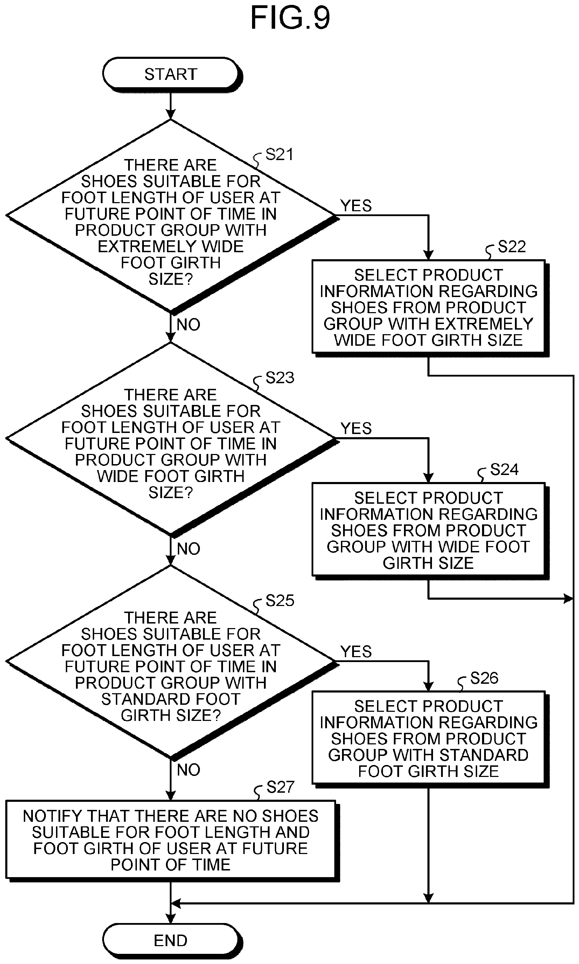

FIG. 9 is a flowchart illustrating a procedure of an operation in which the shoe purchase recommendation device according to the fourth embodiment selects product information regarding shoes whose foot circumference size is one-step closer to a standard size than a size suitable for the foot circumference of a user at a future point of time. Here, it is assumed that four foot circumference sizes of narrow, standard, wide, and extra-wide are set for each shoe. Here, a case where shoes with an extremely wide foot circumference size are suitable for the foot circumference of the user at the future point of time will be described as an example.

In step S21, the product information selection unit 14 determines whether there are shoes suitable for the foot length of the user at a future point of time in a product group with the extremely wide foot circumference size. When there are shoes suitable for the foot length of the user at the future point of time in the product group with the extremely wide foot circumference size, which means "Yes" in step S21, and the product information selection unit 14 selects product information regarding shoes from the product group with the extremely wide foot circumference size in step S22.

When there are no shoes suitable for the foot length of the user at the future point of time in the product group with the extremely wide foot circumference size, which means "No" in step S21, and the product information selection unit 14 determines whether there are shoes suitable for the foot length of the user at the future point of time in the product group with the wide foot circumference size in step S23. When there are shoes suitable for the foot length of the user at the future point of time in the product group with the wide foot circumference size, which means "Yes" in step S23, and the product information selection unit 14 selects product information regarding shoes from the product group with the wide foot circumference size in step S24.

When there are no shoes suitable for the foot length of the user at the future point of time in the product group with the wide foot circumference size, which means "No" in step S23, and the product information selection unit 14 determines whether there are shoes suitable for the foot length of the user at the future point of time in the product group with the standard foot circumference size in step S25. When there are shoes suitable for the foot length of the user at the future point of time in the product group with the standard foot circumference size, which means "Yes" in step S25, and the product information selection unit 14 selects product information regarding shoes from the product group with the standard foot circumference size in step S26.

In this manner, the shoe purchase recommendation device 10D according to the fourth embodiment selects, based on the foot length and the foot circumference of the user, product information regarding shoes with the shoe width suitable for the second foot size in step S22, step S24, or step S26.

When there are no shoes suitable for the foot length of the user at the future point of time in the product group with the standard foot circumference size, which means "No" in step S25, and the product information selection unit 14 notifies a display processing unit 15 that there are no shoes suitable for the foot length of the user at the future point of time in step S27.

The display processing unit 15 notified that there are no shoes suitable for the foot length and the foot circumference of the user at the future point of time displays a message indicating that there is no purchase recommendation product on a purchase recommendation screen 300.

In the above example, the processing when the foot circumference size suitable for the foot circumference of the user at the future point of time is extremely wide has been described. However, when the foot circumference size suitable for the foot circumference of the user at the future point of time is wide or narrow and when there are no shoes suitable for both the foot length and the foot circumference of the user at the future point of time, similarly, product information regarding shoes suitable for the foot length of the user at the future point of time and having a foot circumference size one-step closer to the standard size than the size suitable for the foot length of the user at the future point of time is selected.

In the shoe purchase recommendation device 10D according to the fourth embodiment, when there is no product information regarding shoes suitable for both the foot length and the foot circumference of the user at the future point of time, the product information selection unit 14 selects product information regarding shoes suitable for the foot length of the user at the future point of time and having the foot circumference size one-step closer to the standard size than the size suitable for the foot circumference of the user at the future point of time. Therefore, the shoe purchase recommendation device 10D is less likely to display a message indicating that there are no shoes suitable for the foot length and the foot circumference of the user at the future point of time on the purchase recommendation screen 300.

In each of the above embodiments, the shoe purchase recommendation devices 10A, 10B, 10C, and 10D, which are embodiments of the shoe product information providing device, have been described. However, the shoe product information providing device may be a device that only provides shoe product information without recommending purchase of shoes.

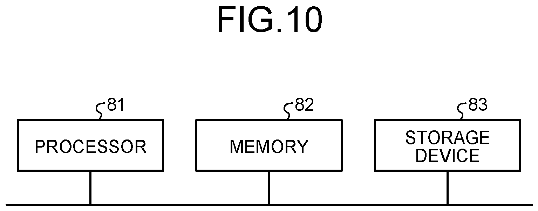

Next, a hardware configuration of each of the shoe purchase recommendation devices 10A, 10B, 10C, and 10D in the above embodiments will be described. FIG. 10 is a diagram illustrating a hardware configuration example of each of the shoe purchase recommendation devices according to the first to fourth embodiments. FIG. 10 illustrates the hardware configuration when the functions of each of the shoe purchase recommendation devices 10A, 10B, 10C, and 10D are implemented by using hardware that executes a program.

Each of the shoe purchase recommendation devices 10A, 10B, 10C, and 10D includes a processor 81 that executes various types of processing, a memory 82 that is a built-in memory, and a storage device 83 that stores information. The processor 81 loads a program stored in the storage device 83 into the memory 82 and executes the program to implement the functions of the foot-size prediction unit 12, the product information selection unit 14, the display processing unit 15, and the growth-end determination unit 16. The user information storage unit 11 and the product information storage unit 13 are implemented by the storage device 83.

Hereinafter, various aspects of the present invention are described.

A shoe product information providing device according to a first aspect includes a user information acquisition unit that acquires user information including a first foot size, the first foot size being a foot size of a user, a user information storage unit that stores the user information acquired by the user information acquisition unit, a foot-size prediction unit that predicts, based on the first foot size of the user stored in the user information storage unit, a second foot size, the second foot size being a foot size of the user at a future point of time, a growth-end determination unit that determines, based on the second foot size predicted by the foot-size prediction unit, whether foot growth of the user has ended at the future point of time, a product information storage unit that stores child product information, the child product information being information regarding a product group of shoes for children, and adult product information, the adult product information being information regarding a product group of shoes for adults, a product information selection unit that selects, based on a result determined by the growth-end determination unit, product information regarding shoes from one of the child product information and the adult product information, and a display processing unit that displays the product information regarding shoes selected by the product information selection unit on a display unit.

According to a shoe product information providing device according to a second aspect, in the shoe product information providing device according to the first aspect, the product information selection unit selects product information regarding shoes suitable for the second foot size.

According to a shoe product information providing device according to a third aspect, in the shoe product information providing device according to the first or second aspect, the product information selection unit selects at least one piece of product information regarding shoes for adults from the adult product information when the growth-end determination unit determines that the foot growth of the user has ended, and the product information selection unit selects at least one piece of product information regarding shoes for children from the child product information when the growth-end determination unit determines that the foot growth of the user has not ended.

According to a shoe product information providing device according to a fourth aspect, in the shoe product information providing device according to the third aspect, the second foot size includes a foot length and a foot circumference of the user at the future point of time, the growth-end determination unit determines that the foot growth of the user has ended at the future point of time when both of following conditions 1 and 2 are satisfied, he growth-end determination unit determines that the foot growth of the user has not ended at the future point of time when at least one of the following conditions 1 and 2 is not satisfied, the condition 1 is that a foot circumference ratio obtained by dividing the foot circumference of the user at the future point of time by the foot length is greater than or equal to a preset threshold, and the condition 2 is that an age of the user at the future point of time is greater than or equal to a predetermined age.

According to a shoe product information providing device according to a fifth aspect, in the shoe product information providing device according to any one of the first to fourth aspects, the product information regarding shoes selected by the product information selection unit includes product information regarding a first pair of shoes and product information regarding a second pair of shoes different in type from the first pair of shoes, and the display processing unit displays, on the display unit, the product information regarding the first pair of shoes and the product information regarding the second pair of shoes selected by the product information selection unit.

According to a shoe product information providing device according to a sixth aspect, in the shoe product information providing device according to the fifth aspect, the first pair of shoes is classified in a first category, and the second pair of shoes is classified in a second category whose use is different from the first category.

According to a shoe product information providing device according to a seventh aspect, in the shoe product information providing device according to the sixth aspect, shoes classified in the first category and shoes classified in the second category are varied in size by applying different last sets from each other, and the product information selection unit includes a plurality of last conversion tables indicating correspondence between a foot size and a shoe size, and selects the product information regarding shoes using the last conversion table matching the last set applied for size variations of each of the shoes in the first category and the shoes in the second category.

According to a shoe product information providing device according to an eighth aspect, in the shoe product information providing device according to any one of the first to seventh aspects, the second foot size includes a foot length and a foot circumference of the user at the future point of time, the child product information and the adult product information each include shoe width information, and the product information selection unit selects, based on the foot length and the foot circumference of the user, product information regarding shoes having a shoe width suitable for the second foot size.

According to a shoe product information providing device according to a ninth aspect, in the shoe product information providing device according to the eighth aspect, when there are no shoes suitable for both the foot length and the foot circumference of the user at the future point of time in the child product information and the adult product information, the product information selection unit selects product information regarding shoes suitable for the foot length of the user at the future point of time and having a shoe width one-step closer to a standard size than a shoe width suitable for the foot circumference of the user at the future point of time.

According to a shoe product information providing device according to a tenth aspect, in the shoe product information providing device according to any one of the first to ninth aspects, the product information regarding shoes selected by the product information selection unit includes an image of the shoes and a uniform resource locator of a website of an electronic commerce operator that sells the shoes, and the display processing unit displays, on the display unit, the image of the shoes selected by the product information selection unit and the uniform resource locator of the website of the electronic commerce operator that sells the shoes selected by the product information selection unit.

A shoe product information providing device according to the present invention has an effect of providing product information regarding shoes in consideration of whether a foot shape of a user is an adult foot shape.

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

## Claims

1. A shoe product information providing device (10A) comprising:
a user information acquisition unit (18) configured to acquire user information (111) including a first foot size (111B), the first foot size being a foot size of a user;
a user information storage unit (11) configured to store the user information acquired by the user information acquisition unit;
a foot-size prediction unit (12) configured to predict, based on the first foot size of the user stored in the user information storage unit, a second foot size, the second foot size being a foot size of the user at a future point of time;
a growth-end determination unit (16) configured to determine, based on the second foot size predicted by the foot-size prediction unit, whether foot growth of the user has ended at the future point of time;
a product information storage unit (13) configured to store child product information (131), the child product information being information regarding a product group of shoes for children, and adult product information(132), the adult product information being information regarding a product group of shoes for adults;
a product information selection unit (14) configured to select, based on a result determined by the growth-end determination unit, product information regarding shoes from one of the child product information and the adult product information; and
a display processing unit (15) configured to display the product information regarding shoes selected by the product information selection unit on a display unit.

2. The shoe product information providing device according to claim 1, wherein the product information selection unit selects product information regarding shoes suitable for the second foot size.

3. The shoe product information providing device according to claim 1 or 2, wherein
the product information selection unit selects at least one piece of product information regarding shoes for adults from the adult product information when the growth-end determination unit determines that the foot growth of the user has ended, and
the product information selection unit selects at least one piece of product information regarding shoes for children from the child product information when the growth-end determination unit determines that the foot growth of the user has not ended.

4. The shoe product information providing device according to claim 3, wherein
the second foot size includes a foot length and a foot circumference of the user at the future point of time,
the growth-end determination unit determines that the foot growth of the user has ended at the future point of time when both of following conditions 1 and 2 are satisfied,
the growth-end determination unit determines that the foot growth of the user has not ended at the future point of time when at least one of the following conditions 1 and 2 is not satisfied,
the condition 1 is that a foot circumference ratio obtained by dividing the foot circumference of the user at the future point of time by the foot length is greater than or equal to a preset threshold, and
the condition 2 is that an age of the user at the future point of time is greater than or equal to a predetermined age.

5. The shoe product information providing device according to claim 1, wherein
the product information regarding shoes selected by the product information selection unit includes product information regarding a first pair of shoes and product information regarding a second pair of shoes different in type from the first pair of shoes, and
the display processing unit displays, on the display unit, the product information regarding the first pair of shoes and the product information regarding the second pair of shoes selected by the product information selection unit.

6. The shoe product information providing device according to claim 5, wherein
the first pair of shoes is classified in a first category, and
the second pair of shoes is classified in a second category whose use is different from the first category.

7. The shoe product information providing device according to claim 6, wherein
shoes classified in the first category and shoes classified in the second category are varied in size by applying different last sets from each other, and
the product information selection unit includes a plurality of last conversion tables (141) indicating correspondence between a foot size and a shoe size, and selects the product information regarding shoes using the last conversion table matching the last set applied for size variations of each of the shoes in the first category and the shoes in the second category.

8. The shoe product information providing device according to claim 1, wherein
the second foot size includes a foot length and a foot circumference of the user at the future point of time,
the child product information and the adult product information each include shoe width information, and
the product information selection unit selects, based on the foot length and the foot circumference of the user, product information regarding shoes having a shoe width suitable for the second foot size.

9. The shoe product information providing device according to claim 8, wherein
when there are no shoes suitable for both the foot length and the foot circumference of the user at the future point of time in the child product information and the adult product information, the product information selection unit selects product information regarding shoes suitable for the foot length of the user at the future point of time and having a shoe width one-step closer to a standard size than a shoe width suitable for the foot circumference of the user at the future point of time.

10. The shoe product information providing device according to claim 1, wherein
the product information regarding shoes selected by the product information selection unit includes an image of the shoes and a uniform resource locator (303) of a website of an electronic commerce operator that sells the shoes, and
the display processing unit displays, on the display unit, the image of the shoes selected by the product information selection unit and the uniform resource locator of the website of the electronic commerce operator that sells the shoes selected by the product information selection unit.

11. A shoe product information providing method comprising:
a user information acquisition step of acquiring user information including data on a foot size of a user;
a foot-size prediction step of predicting, based on the foot size of the user acquired in the user information acquisition step, a foot size of the user at a future point of time;
a growth-end determination step of determining, based on the foot size of the user predicted in the foot-size prediction step, whether foot growth of the user has ended at the future point of time;
a shoe information selection step of selecting, based on a result determined in the growth-end determination step, product information regarding shoes from one of child product information, the child product information being information regarding a product group of shoes for children, and adult product information, the adult product information being information regarding a product group of shoes for adults; and
a display processing step of displaying the product information regarding shoes selected in the shoe information selection step.
